# EUROPEAN PATENT SPECIFICATION

(11) **EP 0 503 388 B1**
(45) Date of publication and mention of the grant of the patent: **18.12.1996**
(21) Application number: 92103353.6
(22) Date of filing: 27.02.1992
(51) Int. Cl.: C07J 7/00, A61K 31/57, C07J 51/00

(54) **Pregna-21-oic acid esters**
Ester von Pregna-21-säure
Esters des acides pregna-21-oics

(30) Priority: 04.03.1991 JP 37155/91
(43) Date of publication of application: 16.09.1992
(73) Proprietor: TEIKOKU SEIYAKU KABUSHIKI KAISHA, Okawa-gun Kagawa-ken (JP); OHTA PHARMACEUTICAL CO., LTD., Ohmiya-shi, Saitama-ken (JP)
(72) Inventor: Suzuki, Toshio, 201-4, Akita-shi, Akita-ken (JP); Kimura, Toshikiro, Okayama-shi, Okayama-ken (JP)
(74) Representative: Kraus, Walter, Dr.

(56) References cited:
- WO-A-87/05028
- FR-A- 2 255 079
- US-A- 4 257 969
- US-A- 4 511 511
- US-A- 4 588 530
- JOURNAL OF STEROID BIOCHEMISTRY vol. 17, no. 3, September 1982, Oxford, GB, B. MANZ et al, pp. 335-342
- DRUGS UNDER EXPERIMENTAL AND CLINICAL RESEARCH vol. 15, no. 11, 1989, S.U. HONG et al, pp. 511-520
- CHEMICAL ABSTRACTS, vol. 109, no. 11, 12 September 1988, Columbus, Ohio, US, abstract no. 86427, H.P. KIM et al
- JOURNAL OF PHARMACY AND PHARMACOLOGY vol. 38, no. 8, August 1986, J. BIRD et al, pp. 589-594
- CHEMICAL ABSTRACTS, vol. 116, no. 15, 13 April 1992, Columbus, Ohio, US, abstract no. 152133, Y. FUJIWARA et al
- CHEMICAL ABSTRACTS, vol. 116, no. 15, 13 April 1992, Columbus, Ohio, US, abstract no. 143484, T. KIMURA et al

## Description

The present invention relates to a novel steroid derivative which has excellent topical anti-inflammatory activity without glucocorticoid activity and further vasoconstrictive activity and is useful as a medicament.

In order to obtain a steroid derivative having a potent, sharp and prolonged action for use as an agent for treating inflammation and alergy, many natural and semi-synthetic steroids have been studied and a variety of steroidal drugs have been developed. At present, the steroidal drugs are considered to be an essential medicament for treating chronic rheumatism and a dermatological disorder as well as inflammation and alergy. In recent years, a steroid derivative having a potent topical anti-inflammatory activity has been desired and a steroid derivative having a potent anti-inflammatory activity has been developed, including dexamethasone valerate, dexamethasone dipropionate, chrobetasole propionate, and the like.

However, the above-mentioned steroid derivatives have not only a potent topical anti-inflammatory activity as a main action but also undesirable systemic side effects of glucocorticoid such as decrease of adrenal secretion, decrease of immunological competence, osteoporosis, full moon-like face due to disorder of lipid or protein metabolism, fatty liver, infantile growth disturbance, and the like.

Accordingly, there has been desired a steroid derivative which has a potent anti-inflammatory activity with high selectivity without showing any glucocorticoidal side effect.

A number of 3-oxo-1,4-pregnadien-21-oates are already known in the state of the art. In particular, the following prior art documents relate to 3-oxo-1,4-pregnadien-21-oates:
D(1) US-A-4511511
D(2) B. MANZ et al. J.Steroid Biochem 17, 335 (1982)
D(3) S.U. HONG et al. Drugs Exptl.Clin.Res. XV, 511 (1989)
D(4) Chem. Abstr. 109, 86427y (1988)
D(5) WO 87/05028
D(6) US-A-4588530
D(7) US-A-4257969
D(8) FR-A-2255079
D(9) J. BIRD et al. J.Pharm.Pharmacol. 38, 589 (1986)

Under the circumstances, the present inventors have prepared various derivatives at the C₂₀-position of methyl (20R/S)-prednisolonate and methyl (20R/S)-dexamethasonate, which are prepared by treating prednisolone or dexamethasone with copper acetate in absolute methanol, and studied pharmacological activities of these derivatives. As a result, the present inventors have found that a novel steroid derivative of the following formula (I) has an extremely potent vasoconstrictive activity. In addition, the present inventors have found that the steroid derivative of the formula (I) has a topical anti-inflammatory activity but does not show an undesirable side effect of glucocorticoids since these compounds of the invention are easily hydrolized with esterase in plasma into corresponding inactive carboxylic acids.

An object of the present invention is to provide a novel steroid derivative having excellent anti-inflammatory activity with high selectivity, that is, no glucocorticoid activity and further excellent vasoconstrictive activity. Another object of the invention is to provide a pharmaceutical composition containing as an active ingredient the steroid compound. These and other objects and advantages of the present invention will be apparent to those skilled in the art from the following description.

The novel steroid compound of the present invention has the following formula (I): 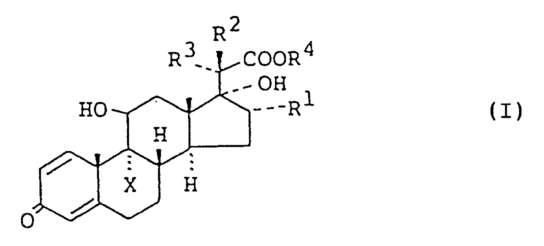 wherein
- (i): X = H, R¹ = H, R² = -OCOC(CH₃)₃, R³ = H, R⁴ = CH₃;
- (ii): X = F, R¹ = CH₃, R² = H, R³ = -OCOCH₃, R⁴ = CH₃; or
- (iii): X = F, R¹ = CH₃, R² = H, R³ = -OCOC(CH₃)₃, R⁴ = CH₃.

The compound (I) of the present invention can be prepared by treating a commercially available steroid having hydroxymethylketone group at the C₁₇-position such as prednisolone, dexamethasone etc. in absolute methanol in the presence of anhydrous copper acetate to convert into a corresponding trihydroxy compound, and conducting acylation, alkylation or silylation of the C₂₀ hydroxy group thereof. Processes for preparing the compound (I) of the present invention are described in detail hereinbelow.

### Process (A) (not claimed)

Process for preparing the compound (I) wherein X = R¹ = R² = H, R³ = acetoxy and R⁴ = methyl and the compound (I) wherein X = R¹ = R³ = H, R² = acetoxy and R⁴ = methyl:

As shown in the following reaction scheme 1, a mixture of prednisolone (II) and anhydrous copper acetate in absolute alcohol is stirred at room temperature to give a methyl ester of a trihydroxy compound (III) as a mixture of two compounds which are diasteromers regarding the C₂₀-position. The compound (III) is then subjected to acetylation reaction with acetic anhydride in the presence of pyridine to give separable two acetyl compounds (1) and (2).

### Reaction scheme 1:

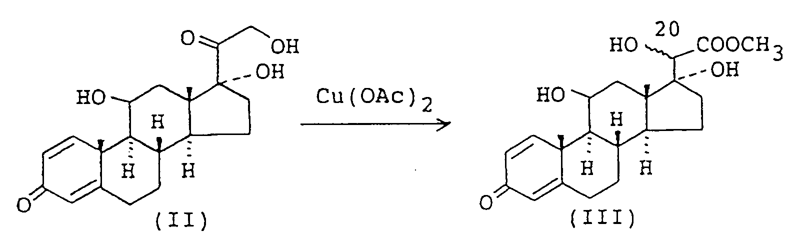

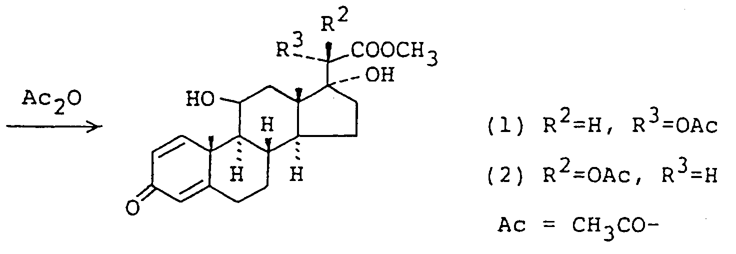

The alcohol used in the above reaction for conversion of the compound (II) into the compound (III) may be any alcohol but a lower alcohol such as methanol and ethanol is preferable in view of solubility. (In case of absolute alcohol used in other processes described hereinbelow, preferred one is a lower alcohol, too) When ethanol is used as the alcohol, a corresponding ethyl ester compound is prepared.

The molar ratio of copper acetate to prednisolone in the first reaction may be in a range of 0.1 to 3 moles of copper acetate to 1 mole of prednisolone. However, a reaction time and an amount of copper acetate are significantly effective on the ratio and yield of the two C₂₀-isomers. That is, when a larger molar ratio of copper acetate to prednisolone (i.e. at least 2 moles of copper acetate to 1 mole of prednisolone) is used and the reaction time is longer (more than 26 days), a ratio of the compound (1) to the compound (2) is increased. On the other hand, when the molar ratio of copper acetate to prednisolone is 0.5 and the reaction time is about 26 days, a yield of the compound (2) and a total yield is increased.

### Process (B):

Process for preparing the compound (I) wherein X = R¹ = R² = H, R³ = OCO-tert-butyl and R⁴ = methyl and the compound (I) wherein X = R¹ = R³ = H, R² = OCO-tert-butyl and R⁴ = methyl:

As shown in the following reaction scheme 2, the compound (1) or (2) prepared in the above process (A) is treated with potassium carbonate in absolute alcohol to give a corresponding trihydroxy compound (IIIa) or (IIIb), respectively. The compound (IIIa) or (IIIb) is then subjected to pivaloylation reaction with pivaloyl chloride in an anhydrous solvent in the presence of a base at room temperature to give a pivaloyl ester compound (3) or (4), respectively.

### Reaction scheme 2:

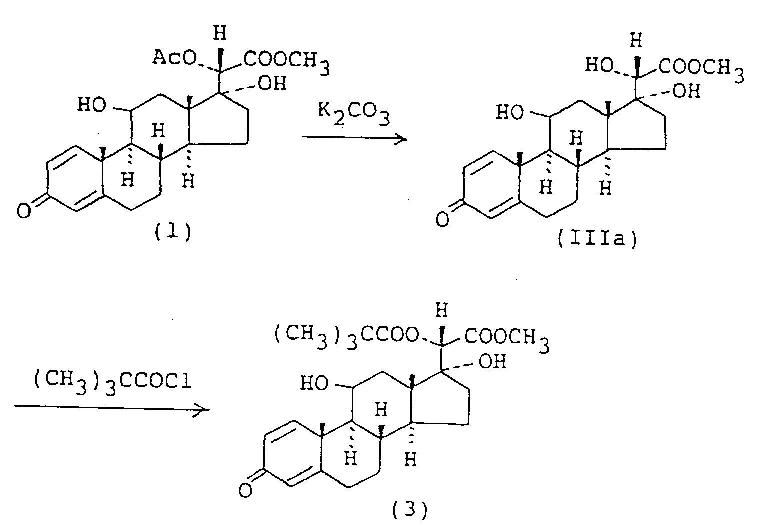

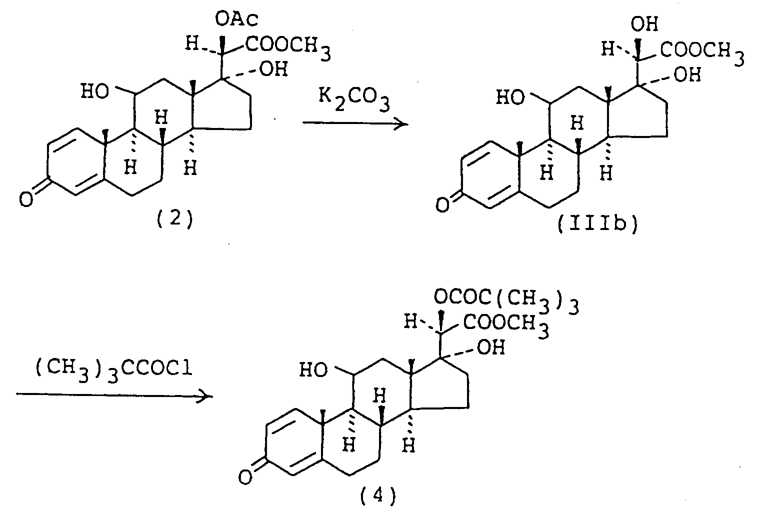

The deacetylation reaction is preferably conducted at a lower temperature (0°C to room temperature) for a short time, because there is a possibility of hydrolysis of the methyl ester. It is sufficient to use potassium carbonate in 1/10 times of an amount to the compound (1) or (2).

The base used in the pivaloylation reaction is preferably a tertiary amine such as trimethylamine, triethylamine, 1,5-diazabicyclo[4.3.0]non-5-ene (DBN), 1,8-diazabicyclo[5.4.0]undec-7-ene (DBU), etc., and an aromatic amine such as pyridine, 4,4-dimethylaminopyridine, etc. The reaction solvent is preferably a solvent which does not affect the reaction such as tetrahydrofuran, chloroform, dichloromethane, dimethylformamide, dimethyl sulfoxide, etc.

### Process (C):

Process for preparing the compound (I) wherein X = F, R¹ = R⁴ = methyl, R² = H and R³ = acetoxy and the compound (I) wherein X = F, R¹ = R⁴ = methyl, R² = acetoxy and R³ = H:

As shown in the following reaction scheme 3, a mixture of dexamethasone (IV) and anhydrous copper acetate in absolute alcohol is stirred at room temperature to give a methyl ester of a trihydroxy compound (V) as a mixture of two compounds which are diasteromers regarding the C₂₀-position. The compound (V) is then subjected to acetylation reaction with acetic anhydride in the presence of pyridine to give separable two acetyl compounds (7) and (8).

### Reaction scheme 3:

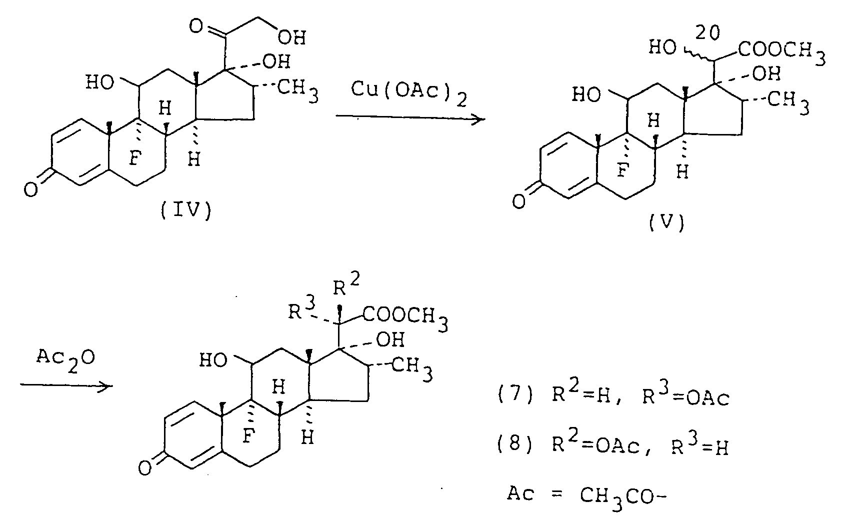

It is preferable that an amount of copper acetate used in the first reaction of this process is in a range of 0.1 to 2.0 equivalents to dexamethasone and the reaction time is around 7 days. Under any condition in this range, the compound (7) is obtained as a main product and the compound (8) is obtained in a small amount.

The alcohol used in the conversion of the compound (IV) into the compound (V) is preferably a lower alcohol such as methanol and ethanol. When ethanol is used as the solvent, a corresponding ethyl ester compound is prepared.

### Process (D) (not claimed)

Process for preparing the compound (I) wherein X = F, R¹ = R⁴ = methyl, R² = H, R³ = OH and the compound (I) wherein X = F, R¹ = R⁴ = methyl, R² = OH and R³ = H:

As shown in the following reaction scheme 4, the acetyl compound (7) or (8) is treated with a catalytic amount of potassium carbonate in an absolute alcohol at room temperature to give a corresponding trihydroxy compound (5) or (6), respectively.

### Reaction scheme 4:

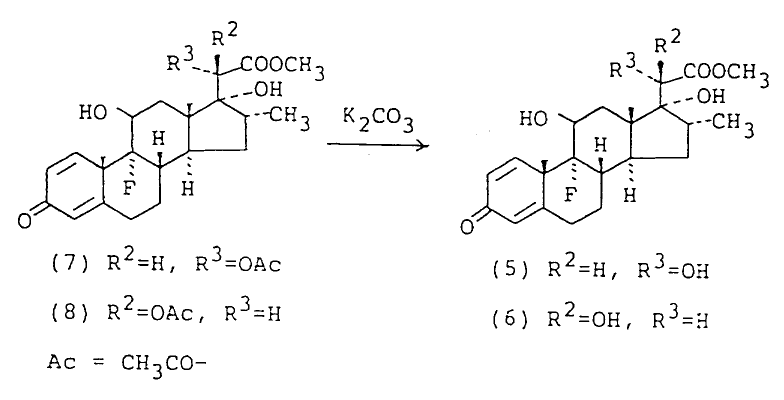

The reaction is preferably conducted at a lower temperature (0°C to room temperature) for a short time, because there is a possibility of hydrolysis of the methyl ester. An amount of potassium carbonate is preferably around 1/10 times of an amount of the compound (7) or (8).

### Process (E):

Process for preparing the compound (I) wherein X = F, R¹ = R⁴ = methyl, R² = H and R³ = t-butylcarbonyloxy and the compound (I) wherein X = F, R¹ = R⁴ = methyl, R² = t-butylcarbonyloxy and R³ = H:

As shown in the following reaction scheme 5, the hydroxy compound (5) or (6) prepared in the above process (D) is subjected to pivaloyl reaction with pivaloyl chloride in an anhydrous solvent in the presence of a base at room temperature to give a corresponding pivaloyl compound (9) or (10), respectively.

### Reaction scheme 5:

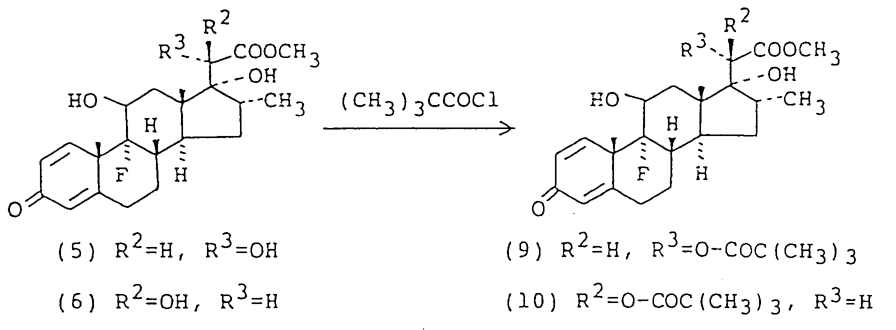

As in the pivaloylation reaction described above, the base used in the pivaloylation reaction is preferably a tertiary amine such as trimethylamine, triethylamine, 1,5-diazabicyclo[4.3.0]non-5-ene (DBN), 1,8-diazabicyclo[5.4.0]undec-7-ene (DBU), etc. The reaction solvent is preferably a solvent which does not affect the reaction such as tetrahydrofuran, chloroform, dichloromethane, dimethylformamide, dimethyl sulfoxide, etc.

Hence, the steroid compound (I) of the present invention can be prepared by the following steps:
Step (a):
   acylating the steroid compound of the following formula: 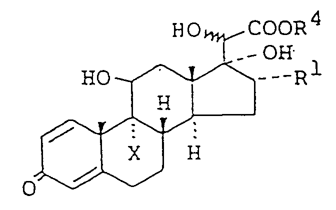 wherein X, R¹ and R⁴ are as defined above, with an acylating agent, or
Step (b):
   acylating the steroid compound of the following formula: 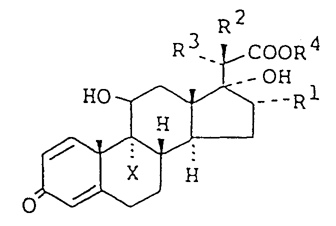 wherein X, R¹ and R⁴ are as defined above, and one of R² and R³ is hydroxyl and the other is hydrogen, with an acylating agent.

The present invention is explained in more detail by means of Examples but should not be construed to be limited thereto.

### Reference Example 1

### Preparation of methyl (20S/R)-acetoxy-11β,17-dihydroxy-3-oxo-1,4-pregnadien-21-oate (1) and (2):

To a solution of Cu(OAc)₂ (1.2 g, 0.007 mole) in absolute methanol (350 ml) is added dropwise a solution of prednisolone (5 g, 0.0139 mole) in absolute methanol (350 ml) while stirring at room temperature. The reaction mixture is stirred at room temperature for 26 days. After adding an aqueous solution (70 ml) of EDTA (2.8 g, 0.0075 mole), the solvent is distilled off under reduced pressure. The obtained residue is extracted with chloroform and the chloroform layer is washed with brine, dried over anhydrous MgSO₄ and the solvent is distilled off to give a crude product (5.5 g). The obtained product is then subjected to silica-gel column chromatography with CHCl₃/MeOH (99/1, v/v) as an eluent to give methyl (20S/R)-11β,17,20-trihydroxy-3-oxo-1,4-pregnadien-21-oate (3.21 g) as a mixture of two compounds which are diastereomers regarding the C₂₀-position. These compounds are difficult to separate, and hence, are used in the next acetylation reaction without purification.

To a solution of the above mixture (1 g) of methyl ester compounds in anhydrous pyridine (3 ml) is added acetic anhydride (3 ml) and the mixture is stirred at room temperature for 20 hours. After completion of the reaction, a small amount of water (1 ml) is added to the reaction mixture to decompose acetic anhydride, and acetic acid and pyridine are distilled off under reduced pressure. The residue is dissolved in chloroform and the chloroform layer is washed with brine, dried over anhydrous MgSO₄ and the solvent is distilled off. The obtained residue is then subjected to silica-gel column chromatography with CHCl₃/MeOH (99/1, v/v) as an eluent to give two colorless caramels (2) (303 mg, 16.2 % from prednisolone) and (1) (380 mg, 20.3 % from prednisolone). These two caramels are recrystallized from methanol to give the compound (2) as colorless needles and the compound (1) as colorless prisms, each compound having the following properties.

### Compound (1) [X = R¹ = R² = H, R³ = acetoxy]

Melting point: 203 to 204°C
[α]²²_{D}: + 44.7° (c = 0.3, CHCl₃)
IRₘₐₓ (CHCl₃) cm⁻¹: 3575, 3550 - 3200 (OH), 1749, 1600 (CO), 1620, 1605 (C=C)
¹H-NMR: δppm (CDCl₃): 1.05 (3H, s, CH₃), 1.45 (3H, s, CH₃), 2.17 (3H, s, COCH₃), 3.73 (3H, s, CO₂CH₃), 5.08 (1H, s, C₂₀-H), 4.30 - 4.53 (1H, m, C₁₁-H), 5.97 (1H, brs, C₄-H), 6.21 (1H, dd, J = 10, 2 Hz, C₂-H), 7.20 (1H, d, J = 10 Hz, C₁-H)

### Compound (2) [X = R¹ = R³ = H, R² = acetoxy]

Melting point: 236 to 238°C
[α]²²_{D}: + 69.3° (c = 0.3, CHCl₃)
IRₘₐₓ (CHCl₃) cm⁻¹: 3575, 3550 - 3200 (OH), 1749, 1600 (CO), 1620, 1605 (C=C)
¹H-NMR: δppm (CDCl₃): 1.23 (3H, s, CH₃), 1.47 (3H, s, CH₃) , 2.15 (3H, s, COCH₃) , 3.75 (3H, s, CO₂CH₃), 4.28 - 4.67 (1H, m, C₁₁-H), 5.07 (1H, s, C₂₀-H), 5.98 (1H, brs, C₄-H), 6.12 (1H, dd, J = 10, 2Hz, C₂-H), 7.20 (1H, d, J = 10Hz, C₁-H)

### Example 1

### Preparation of (20R)-11β,17-dihydroxy-3-oxo-20-pivaloyloxy-1,4-pregnadien-21-oate (4):

To a solution of the compound (2) obtained in Reference Example 1 (982 mg) in absolute methanol (10 ml) is added K₂CO₃ (98 mg) and the mixture is stirred at room temperature for 10 minutes. Chloroform is added to the reaction mixture and the mixture is washed with brine and dried over anhydrous MgSO₄. The solvent is distilled off and the obtained residue is then subjected to silica-gel column chromatography with CHCl₃/MeOH (99/1, v/v) as an eluent to give colorless caramel. This substance is recrystallized from methanol to give methyl (20R)-11β,17,20-trihydroxy-3-oxo-1,4-pregnadien-21-oate (IIIb) (750 mg) as colorless prisms (m.p. 263 to 264°C).

To a solution of the above trihydroxy compound (IIIb) (50 mg, 0.000128 mole) and dimethylaminopyridine (47 mg, 0.000384 mole) in anhydrous CH₂Cl₂ is added dropwise pivaloyl chloride (39.5 µl, 0.00032 mole). The reaction mixture is stirred at room temperature for 22 hours. After completion of the reaction, diethyl ether is added to the reaction mixture and the mixture is washed with a small amount of water and dried over anhydrous MgSO₄, and then the solvent is distilled off. The obtained residue is then subjected to silica-gel column chromatography with CHCl₃/MeOH (99/1, v/v) to give a pivaloyl ester compound [54.6 mg, 82.3 % from the acetyl compound (1)] having the following properties as colorless caramel.
[α]²⁰_{D}: + 34.6° (c = 0.44, CHCl₃)
IRₘₐₓ (CHCl₃) cm⁻¹: 1740, 1665 (CO), 1620, 1605 (C=C)
¹H-NMR: δppm (CDCl₃): 1.23 (3H, s), 1.43 (3H, s), 3.73 (3H, s), 4.27 - 4.57 (1H, m), 6.0 (1H, s), 6.2 (1H, dd, J = 9, 1.5 Hz), 7.20 (1H, d, J = 9 Hz)

### Example 2

### Preparation of methyl (20S/R)-acetoxy-11β,17-dihydroxy-9α-fluoro-16α-methyl-3-oxo-1,4-pregnadien-21-oate (7) and (8):

To a solution of Cu(OAc)₂ (231 mg, 0.00128 mole) in absolute methanol (80 ml) is added dropwise a solution of dexamethanone (1 g, 0.00255 mole) in absolute methanol (80 ml) while stirring at room temperature. The reaction mixture is stirred at room temperature for 7 days. After adding an aqueous solution (15 ml) of EDTA (550 mg, 0.0026 mole), the solvent is distilled off under reduced pressure. The obtained residue is extracted with chloroform and the chloroform layer is washed with brine, dried over anhydrous MgSO₄ and the solvent is distilled off to give a crude product (1.1 g). The obtained product is then subjected to silica-gel column chromatography with CHCl₃/MeOH (99/1, v/v) as an eluent to give methyl (20S/R)-9α-fluoro-16α-methyl-11β,17,20-trihydroxy-3-oxo-1,4-pregnadien-21-oate (589 mg, 54.8 %) as a mixture of two compounds which are diastereomers regarding the C₂₀-position. These compounds are difficult to separate, and hence, are used in the next acetylation reaction without purification.

To a solution of the above mixture (500 mg) of methyl ester compounds in anhydrous pyridine (2 ml) is added acetic anhydride (2 ml) and the mixture is stirred at room temperature for 20 hours. After completion of the reaction, a small amount of water (1 ml) is added to the reaction mixture to decompose acetic anhydride, and acetic acid and pyridine are distilled off under reduced pressure. The residue is dissolved in chloroform and the chloroform layer is washed with brine, dried over anhydrous MgSO₄ and the solvent is distilled off. The obtained residue is then subjected to silica-gel column chromatography with CHCl₃/MeOH (99/1, v/v) as an eluent to give two colorless caramel products (8) (171.9 mg, 17.1 % from dexamethasone) and (7) (239.7 mg, 23.8 % from dexamethasone). These two products are recrystallized from methanol/hexane to give the compound (8) as colorless needles and the compound (7) as colorless prisms, each compound having the following properties.

### Compound (7) [X = F, R¹ = methyl, R² = H, R³ = acetoxy]

Melting point: 263 to 265°C
[α]²⁵_{D}: + 49.4° (c = 0.24, CHCl₃)
IRₘₐₓ (CHCl₃) cm⁻¹: 1740, 1665 (CO), 1625, 1605 (C=C)
¹H-NMR: δppm (CDCl₃): 0.83 (3H, d, J = 7Hz), 1.10 (12H, s), 1.53 (3H, s), 2.10 (3H, s), 3.70 (3H, s), 4.10 - 4.53 (1H, m), 4.90 (1H, s), 6.03 (1H, s), 6.23 (1H, dd, J = 9, 1.5 Hz), 7.13 (1H, d, J = 9 Hz)

### Compound (8) [X = F, R¹ = methyl, R² = acetoxy, R³ = H]

Melting point: 235 to 238°C
[α]²⁵_{D}: + 30.6° (c = 0.22, CHCl₃)
IRₘₐₓ (CHCl₃) cm⁻¹: 1744, 1665 (CO), 1624, 1605 (C=C)
¹H-NMR: δppm (CDCl₃): 1.10 (3H, d, J = 7 Hz), 1.27 (12H, s), 1.53 (3H, s), 2.10 (3H, s), 3.70 (3H, s), 4.10 - 4.53 (1H, m), 5.07 (1H, s), 6.03 (1H, s), 6.23 (1H, dd, J = 9, 1.5 Hz), 7.10 (1H, d, J = 9 Hz)

### Reference Example 2

### Preparation of methyl (20S)-9α-fluoro-16α-methyl-11β,17,20-trihydroxy-3-oxo-1,4-pregnadien-21-oate (5):

To a solution of the compound (7) obtained in Example 4 (163.6 mg) in absolute methanol (4 ml) is added K₂CO₃ (22 mg) and the mixture is stirred at room temperature for 30 munites. Chloroform is added to the reaction mixture and the mixture is washed with brine and dried over anhydrous MgSO₄. The solvent is distilled off and the obtained residue is then recrystallised from MeOH/hexane to give the compound (5) (145 mg, 97.7 %) having the following properties as colorless prisms.
Melting point: 145 - 147°C
[α]²⁴_{D}: + 20.2° (c = 0.22, CHCl₃)
IRₘₐₓ (CHCl₃) cm⁻¹: 1732 (CO), 1624 - 1605 (C=C)
¹H-NMR: δppm (CDCl₃): 0.87 (3H, d, J = 7 Hz), 1.20 (12H, s), 1.53 (3H, s), 3.73 (3H, s), 4.20 - 4.33 (1H, m), 6.07 (1H, s), 6.27 (1H, dd, J = 9, 1.5 Hz), 7.17 (1H, d, J = 9 Hz)

### Example 3

### Preparation of methyl (20S)-11β,17-dihydroxy-9α-fluoro-16α-methyl-3-oxo-20-pivaloyloxy-1,4-pregnadien-21-oate (9):

To a solution of the above trihydroxy compound (5) (50 mg, 0.000123 mole) and dimethylaminopyridine (39 mg, 0.00032 mole) in anhydrous dichloromethane is added dropwise pivaloyl chloride (36 µl, 0.000295 mole). The reaction mixture is stirred at room temperature for 22 hours. After completion of the reaction, diethyl ether is added to the reaction mixture and the mixture is washed with a small amount of water and dried over anhydrous MgSO₄, and then the solvent is distilled off. The obtained residue is then subjected to silica-gel column chromatography with CHCl₃/MeOH (99/1, v/v) to give a pivaloyl ester compound (9) (59 mg, 98.4 %) having the following properties as colorless caramel.
[α]²³_{D}: + 35.7° (c = 0.20, CHCl₃)
IRₘₐₓ (CHCl₃) cm⁻¹: 1740, 1660 (CO), 1620, 1605 (C=C)
¹H-NMR: δppm (CDCl₃): 0.83 (3H, d, J = 7 Hz), 1.08 (3H, s), 1.23 (9H, s), 1.50 (3H, s), 3.67 (3H, s), 4.50 - 4.13 (2H, m), 4.85 (1H, s), 6.0 (1H, s), 6.20 (1H, dd, J = 9, 1.5 Hz), 7.30 (1H, d, J = 9 Hz)

### Vasoconstrictive activity

The compounds of the present invention were tested for the vasoconstrictive activity in the following manner.

Each of the compounds (4), (7) and (9) of the present invention was mixed in a white petrolatum base (white petrolatum : liquid paraffin = 9 : 1) at a concentration of 0.1 % by weight for use as a test sample. Commercially available steroidal medicaments (ointments of prednisolone and dexamethasone) were used at a concentration of 0.1 % by weight of the steroid as a reference sample. A commercially available ointment base was used as a control.

To one portion of the back of volunteers (10 healthy adult males) was applied each sample. About 20 mg of each sample was put in an aluminum part of fin chamber, which was applied. Two hours and four hours after the application, samples were simultaneously removed. After removal, each applied portion of the back was observed for skin paleness after 2 and 4 hours. The result was evaluated based on the following criteria
- (-): Paleness was not observed.
- (±): Paleness was slightly observed.
- (+): Paleness was clearly observed.

Table 1 shows the results of the compounds (4), (7) and (9) of the present invention as well as reference and control.

In Table 1, the results are shown in % of the number of subject which showed (+) or (±) to the total number of subject.

**Table 1**

| Sample | Application time (hr.) | 2 hours after removal | | 4 hours after removal | |
|---|---|---|---|---|---|
| | | + | ± | + | ± |
| Ointment base | 2 | 0 | 0 | 0 | 0 |
| " | 4 | 0 | 0 | 0 | 0 |
| Compound (4) | 2 | 0 | 10 | 0 | 70 |
| " | 4 | 20 | 70 | 40 | 100 |
| Compound (7) | 2 | 0 | 10 | 0 | 50 |
| " | 4 | 30 | 70 | 50 | 80 |
| Compound (9) | 2 | 0 | 0 | 0 | 0 |
| " | 4 | 0 | 50 | 10 | 90 |
| Prednisolone | 2 | 0 | 0 | 0 | 0 |
| " | 4 | 0 | 0 | 0 | 0 |
| Dexamethasone | 2 | 0 | 0 | 0 | 10 |
| " | 4 | 0 | 10 | 0 | 20 |

As shown in the above results, the compounds of the present invention (4), (7) and (9), have an excellent pharmacological activity.

## Claims (Claims for the following Contracting State(s): BE, CH, DE, FR, GB, IT, LI, NL, SE)

1. A steroid derivative of the formula (I): wherein 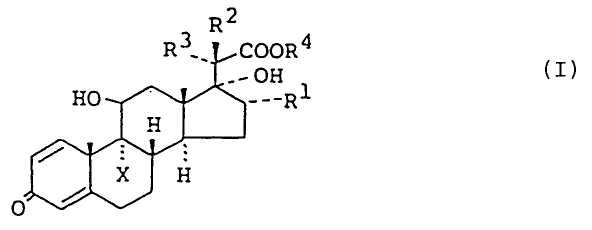
(i) X = H, R¹ = H, R² = -OCOC(CH₃)₃, R³ = H, R⁴ = CH₃;
(ii) X = F, R¹ = CH₃, R² = H, R³ = -OCOCH₃, R⁴ = CH₃; or
(iii) X = F, R¹ = CH₃, R² = H, R³ = -OCOC(CH₃)₃, R⁴ = CH₃.

2. A process for preparing a steroid derivative of the formula (I): 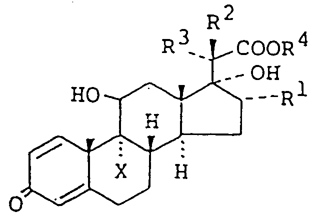 wherein X, R¹, R², R³, R⁴ and R are defined as in claim 1, which comprises the following steps:
Step (a):
acylating the steroid compound of the following formula: 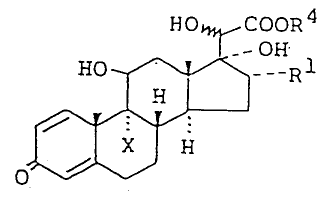 wherein X, R¹ and R⁴ are as defined above, with an acylating agent, or
Step (b):
acylating the steroid compound of the following formula: 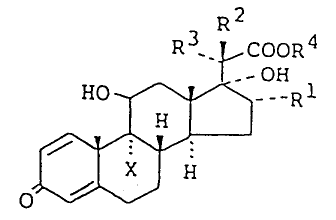 wherein X, R¹ and R⁴ are as defined above, and one of R² and R³ is hydroxyl and the other is hydrogen, with an acylating agent.

## Claims (Claims for the following Contracting State(s): ES)

1. A process for preparing a steroid derivative of the formula (I): 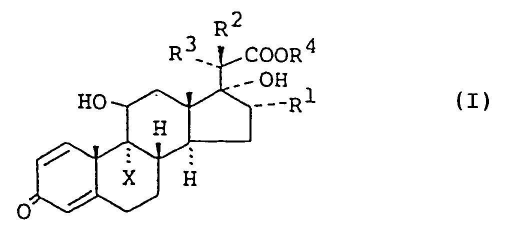 wherein
(i) X = H, R¹ = H, R² = -OCOC(CH₃)₃, R³ = H, R⁴ = CH₃;
(ii) X = F, R¹ = CH₃, R² = H, R³ = -OCOCH₃, R⁴ = CH₃; or
(iii) X = F, R¹ = CH₃, R² = H, R³ = -OCOC(CH₃)₃, R⁴ = CH₃,
which comprises the following steps:
Step (a):
acylating the steroid compound of the following formula: 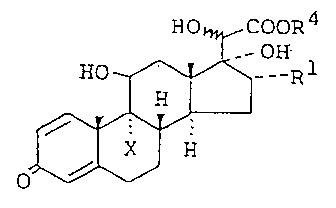 wherein X, R¹ and R⁴ are as defined above, with an acylating agent, or
Step (b):
acylating the steroid compound of the following formula: 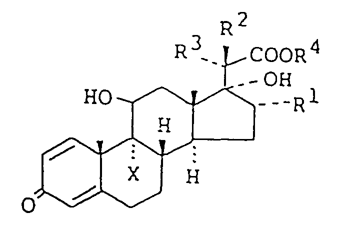 wherein X, R¹ and R⁴ are as defined above, and one of R² and R³ is hydroxyl and the other is hydrogen, with an acylating agent.

## Patentansprüche (Patentansprüche für folgende(n) Vertragsstaat(en): BE, CH, DE, FR, GB, IT, LI, NL, SE)

1. Steroidderivat der Formel (I): 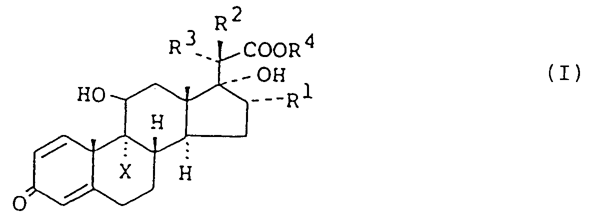 worin
(i) X = H, R¹ = H, R² = -OCOC(CH₃)₃, R³ = H, R⁴ = CH₃ ;
(ii) X = F, R¹ = CH₃, R² = H, R³ = -OCOCH₃, R⁴ = CH₃; oder
(iii) X = F, R¹ = CH₃, R² = H, R³ = -OCOC(CH₃)₃, R⁴ = CH₃.

2. Verfahren zur Herstellung eines Steroidderivats der Formel (I): 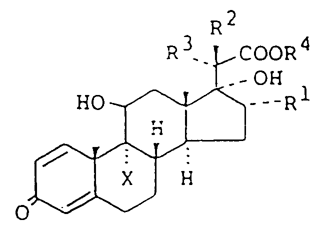 worin X, R¹, R², R³, R⁴ und R die in Anspruch 1 gegebenen Definitionen besitzen, umfassend die folgenden Stufen:
Stufe (a):
Acylierung der Steroidverbindung der folgenden Formel: 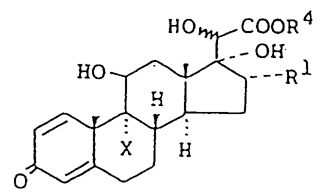 worin X, R¹ und R⁴ die oben gegebenen Definitionen besitzen, mit einem Acylierungsmittel, oder
Stufe (b):
Acylierung der Steroidverbindung der folgenden Formel: 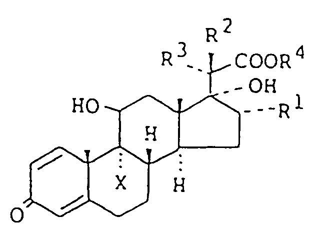 worin X, R¹ und R⁴ die oben gegebenen Definitionen besitzen und einer von R² und R³ Hydroxyl und der andere Wasserstoff bedeutet, mit einem Acylierungsmittel.

## Patentansprüche (Patentansprüche für folgende(n) Vertragsstaat(en): ES)

1. Verfahren zur Herstellung eines Steroidderivats der Formel (I): 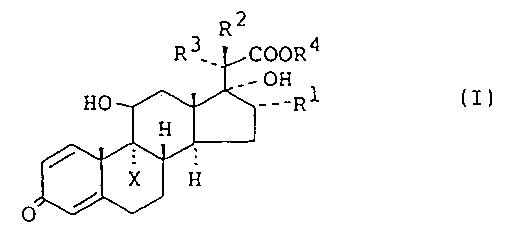 worin
(i) X = H, R¹ = H, R² = -OCOC(CH₃)₃, R³ = H, R⁴ = CH₃ ;
(ii) X = F, R¹ = CH₃, R² = H, R³ = -OCOCH₃, R⁴ = CH₃; oder
(iii) X = F, R¹ = CH₃, R² = H, R³ = -OCOC(CH₃)₃, R⁴ = CH₃,
welches die folgenden Stufen umfaßt:
Stufe (a):
Acylierung der Steroidverbindung der folgenden Formel: 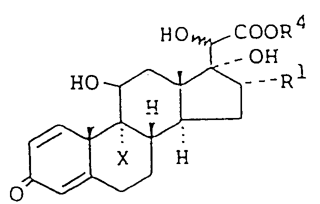 worin X, R¹ und R⁴ die oben gegebenen Definitionen besitzen, mit einem Acylierungsmittel, oder
Stufe (b):
Acylierung der Steroidverbindung der folgenden Formel: 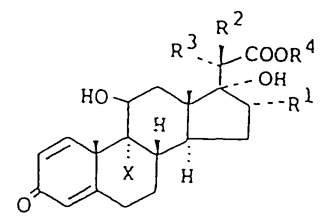 worin X, R¹ und R⁴ die oben gegebenen Definitionen besitzen und einer von R² und R³ Hydroxyl und der andere Wasserstoff bedeutet, mit einem Acylierungsmittel.

## Revendications (Revendications pour l'(les) Etat(s) contractant(s) suivant(s): BE, CH, DE, FR, GB, IT, LI, NL, SE)

1. Dérivé stéroïde de formule (I) : 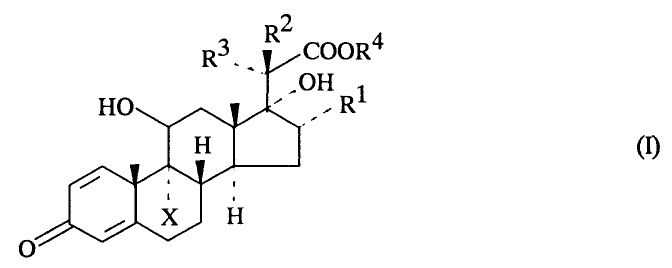 dans laquelle
(i) X = H, R¹ = H, R² = -OCOC(CH₃)₃, R³ = H, R⁴ = CH₃ ;
(ii) X = F, R¹ = CH₃, R² = H, R³ = -OCOCH₃, R⁴ = CH₃ ; ou
(iii) X = F, R¹ = CH₃, R² = H, R³ = -OCOC(CH₃)₃, R⁴ = CH₃.

2. Procédé de préparation d'un dérivé stéroïde de formule (I) : 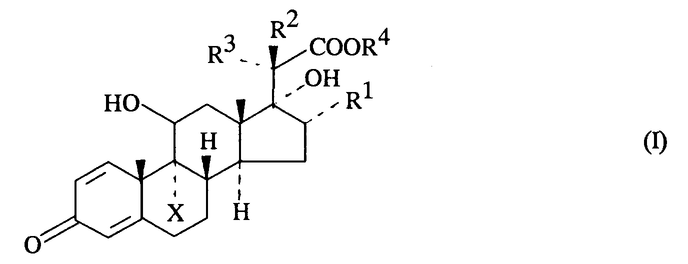 dans laquelle X, R¹, R², R³, R⁴ et R sont définis comme dans la revendication 1, qui comprend les étapes suivantes :
Etape (a) :
acylation du composé stéroïde de formule suivante : 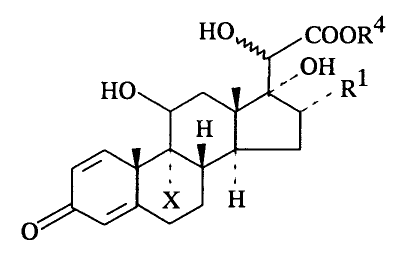 dans laquelle X, R¹ et R⁴ sont tels que défini ci-dessus, avec un agent acylant, ou
Etape (b) :
Acylation du composé stéroïde de formule suivante : 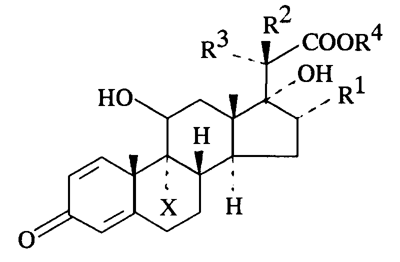 dans laquelle X, R¹ et R⁴ sont tels que définis ci-dessus, et l'un parmi R² et R³ est hydroxyle et l'autre est l'hydrogène, avec un agent acylant.

## Revendications (Revendications pour l'(les) Etat(s) contractant(s) suivant(s): ES)

1. Procédé de préparation d'un dérivé stéroïde de formule (I) : 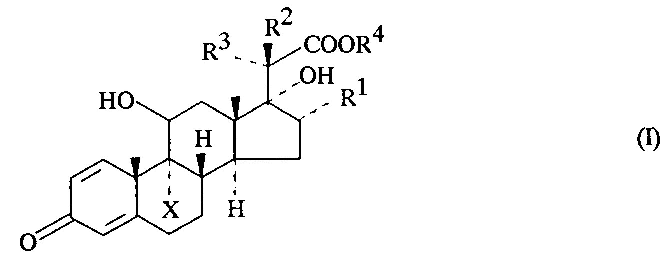 dans laquelle
(i) X = H, R¹ = H, R² = -OCOC(CH₃)₃, R³ = H, R⁴ = CH₃ ;
(ii) X = F, R¹ = CH₃, R² = H, R³ = -OCOCH₃, R⁴ = CH₃ ; ou
(iii) X = F, R¹ = CH₃, R² = H, R³ = -OCOC(CH₃)₃, R⁴ = CH₃,
qui comprend les étapes suivantes :
Etape (a) :
Acylation du composé stéroïde de formule suivante : 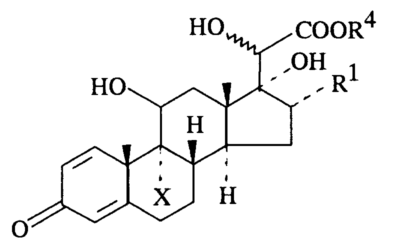 dans laquelle X, R¹ et R⁴ sont tels que défini ci-dessus, avec un agent acylant, ou
Etape (b) :
Acylation du composé stéroïde de formule suivante : 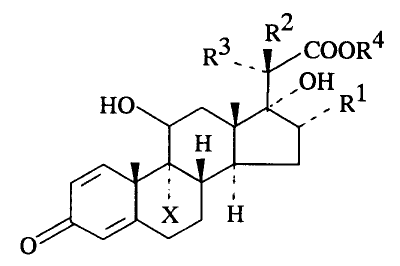 dans laquelle X, R¹ et R⁴ sont tels que définis ci-dessus, et l'un parmi R² et R³ est hydroxyle et l'autre est l'hydrogène, avec un agent acylant.
